# EUROPEAN PATENT APPLICATION

(11) **EP 3 392 335 A1**
(43) Date of publication of application: **24.10.2018**
(21) Application number: 16875595.7
(22) Date of filing: 12.12.2016
(51) Int. Cl.: C12N 5/071

(54) **METHOD FOR CULTURING ADHERENT CELLS**

(30) Priority: 16.12.2015 JP 2015244753
(71) Applicant: Zeon Corporation, Tokyo 100-8246 (JP)
(72) Inventor: KUSABIRAKI, Kazuki, Tokyo 100-8246 (JP); ICHIMURA, Naoya, Tokyo 100-8246 (JP)
(74) Representative: Maiwald Patent- und Rechtsanwaltsgesellschaft mbH
(86) International application number: PCT/JP2016/086930
(87) International publication number: WO 2017/104618

(57) **Abstract**

The invention is a method for culturing adherent cells, culturing the adherent cells in a liquid medium in an alicyclic structure-containing polymer culture vessel, releasing the cells from the culture vessel by liquid flow without adding a protease, and suspending the cultured cells in a liquid medium. The cells can be easily released by a small force such as pipetting because the cells adhere loosely to the alicyclic structure-containing polymer culture vessel.

## Description

### TECHNICAL FIELD

The present invention relates to a method for culturing adherent cells, which allows suspension of cultured cells in a medium only by mild physical actions such as pipetting and vortexing without using a proteinase such as trypsin.

### BACKGROUND ART

For culture of adherent cells, an operation in which when the cells reach a confluent state, the cells adhered to the bottom surface of a cell culture vessel are released by a proteinase such as trypsin and transferred to another vessel has been conventionally adopted. However, due to the problems of damage to the cells by trypsin, virus infection through animals or the like resulting in trypsin, etc., cell culture methods without using proteinases have been investigated (Patent Documents 1 to 4).

However, although all of these methods allowed reduced damages to cells, productivity was poor, e.g. with a further culturing step required. Besides, a method of physically releasing cells from a bottom surface of a culture vessel using a scraper or the like has also been carried out, but there is concern that stress on cells is too great.

Incidentally, Patent Document 5 reports that, in cell culture using a cycloolefin resin vessel, cell proliferation is improved compared to the case of using a polystyrene vessel. In Examples, it is shown that the proliferation of hematopoietic cells other than adherent cells is improved under an environment where proteins such as anti-CD3 antibody and Retronectin are coated.

### CITATION LIST

### PATENT LITERATURE

PTL 1: Japanese Patent Laid-Open No. 5-091872
PTL2: Japanese Patent Laid-Open No. 5-192138
PTL 3: Japanese Patent Laid-Open No. 7-313151
PTL 4: Japanese Patent Laid-Open No. 2012-235764
PTL 5: Japanese Patent Laid-Open No. 2008-048653

### SUMMARY OF INVENTION

### TECHNICAL PROBLEM

The invention has been made in view of the above situation. An object of the present invention is to provide a method for culturing adherent cells, which allows suspension of cultured cells in a medium only by mild physical actions such as pipetting or vortexing without using a proteinase such as trypsin.
It relates to a method.

### SOLUTION TO PROBLEM

To solve the above problem, the inventors investigated a method capable of more easily transferring cultured adherent cells to another vessel. As a result, the inventors has found that when an alicyclic structure-containing polymer culture vessel is used as a culture vessel, damage to the cells can be reduced only by pipetting operation which has been normally carried out in a release process after treating the adherent cells with protease such as trypsin, without treatment with a protease such as trypsin, the cells can be transferred to another vessel, and furthermore cell loss is very small. This finding has led to the completion of the invention.

One aspect of the invention provides a method for culturing adherent cells, characterized in that the adherent cells are cultured in a liquid medium in an alicyclic structure-containing polymer culture vessel, the cells are released from the culture vessel by liquid flow without adding a protease, and the cultured cells are suspended in a liquid medium.

In the present invention, the adherent cells are preferably CHO cells. In addition, a water contact angle of the bottom surface in contact with the cells in the alicyclic structure-containing polymer culture vessel is preferably 85° to 100°.

### BRIEF DESCRIPTION OF DRAWINGS

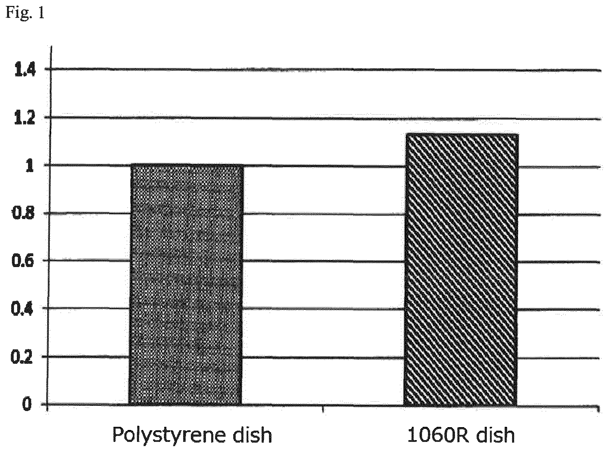

Figure 1 illustrates a graph showing a viable cell count at a time when the second subculture has been finished.

### DESCRIPTION OF EMBODIMENTS

The present invention relates to a method for culturing adherent cells, characterized in that the adherent cells are cultured in a liquid medium in an alicyclic structure-containing polymer culture vessel, the cells are released from the culture vessel by liquid flow without adding a protease, and the cultured cells are suspended in a liquid medium.

That is, in the method according to the invention, when adherent cells are cultured in an alicyclic structure-containing polymer vessel, the cultured cells can be easily transferred to another vessel.

The adherent cells according to the present invention are not particularly limited, and may be arbitrarily selected taking account of the object (intended use). In the present invention, the adherent cells may be either adherent cells themselves or cells derived from the adherent cells. The term "adherent cells themselves" used herein refers to cells that may adhere to an extracellular matrix under normal culture conditions to survive and grow. The adherent cells themselves are also referred to as "anchorage-dependent cell". The term "cells derived from adherent cells" used herein refers to cells that are derived from adherent cells and can survive and grow without adhering to an extracellular matrix due to an external factor applied to the adherent cells (e.g., cells obtained by culturing (acclimating) adherent cells so as to be able to survive and grow even in a suspended state).

Examples of adherent cells include host cells used for gene manipulation, cells for growing, recovering and producing viruses for a vaccine formulation and a gene introduction formulation, various stem cells, non-hematopoietic cells differentiated and induced from stem cells, and the like, such as CHO (Chinese hamster ovary) cells, VERO cells, NIH 3T3 cells and HEK 293 cells.

Also, in the present invention, the adherent cells may be adherent cells in which a foreign gene can be expressed by transduction into these adherent cells with a vector such as a phage and a plasmid, etc.

The foreign gene may be arbitrarily selected taking account of the object (intended use). Specific examples of the foreign gene include a gene that encodes a physiologically active protein (e.g., cytokine and hormone) such as erythropoietin (EPO), interferon (interferon α, interferon β, and interferon γ), granulocyte-colony stimulating factor (G-CSF), interleukin, granulocytic-macrophage colony-stimulating factor (GM-CSF), human-growth hormone, insulin, glucagon (HGF), blood coagulation factor VIII, and a human antibody.

Among these adherent cells, the CHO cells are preferable from the viewpoint of providing more excellent effects according to the present invention,

A liquid medium is used when culturing the cells.

A liquid medium is normally used, which has a pH buffer action, has an osmotic pressure suitable for the cells, includes nutritional ingredients for the cells, and does not have toxicity to the cells.

Examples of components that provide a pH buffer action to the liquid medium include Tris hydrochloride, various phosphates, various carbonates and the like.

The osmotic pressure of the liquid medium is normally adjusted using an aqueous solution that includes potassium ions, sodium ions, calcium ions, glucose, and the like at an adjusted concentration so that the osmotic pressure of the liquid medium is almost equal to that of the cells. Specific examples of such an aqueous solution include physiological saline such as phosphate buffered saline, Tris-buffered saline, and HEPES-buffered saline; a Ringer's solution such as Ringer's lactate solution, Ringer's acetate solution, and Ringer's bicarbonate solution; and the like.

Examples of the nutritional ingredients for the cells include an amino acid, a nucleic acid, a vitamin, a mineral, and the like.

A commercially-available product such as RPMI-1640, HAM, α-MEM, DMEM, EMEM, F-12, F-10, and M-199 may be used as the liquid medium.

An additive may be added to the liquid medium. Examples of the additive to be used include an inducer such as a protein; a low-molecular-weight compound having differentiation-inducing activity; a peptide; a mineral; a metal; a vitamin component; a ligand, an agonist and an antagonist that act on a cell surface acceptor; a ligand, an agonist and an antagonist that act on a nuclear receptor; an extracellular matrix such as collagen and fibronectin; part of the extracellular matrix, or a compound that imitates the extracellular matrix; a component that acts on a protein that is involved in a cell signaling pathway; a component that acts on a primary or secondary metabolism enzyme within the cells; a component that affects intranuclear or intramitochondrial gene expression; DNA and RNA that can be introduced into the cells in combination with a virus vector or the like; and the like.

These additives may be used either alone or in combination.

The cell culture conditions are not particularly limited. The cell culture conditions may be appropriately determined taking account of the cells and the object.

For example, the cells may be cultured using a humidified incubator that contains carbon dioxide at a concentration of about 5%, and is maintained at a constant temperature within the range from 20°C to 37°C.

The alicyclic structure-containing polymer culture vessel used in connection with the invention is obtained by forming an alicyclic structure-containing polymer so as to have an arbitrary shape.

The term "alicyclic structure-containing polymer" used herein refers to a resin that includes an alicyclic structure in either or both of the main chain and the side chain. It is preferable that the alicyclic structure-containing polymer include an alicyclic structure in the main chain from viewpoint of mechanical strength, heat resistance, and the like.

Examples of the alicyclic structure include a saturated cyclic hydrocarbon (cycloalkane) structure, an unsaturated cyclic hydrocarbon (cycloalkene) structure, and the like. It is preferable that the alicyclic structure-containing polymer contains a cycloalkane structure or a cycloalkene structure from the viewpoint of mechanical strength, heat resistance, and the like. It is most preferable that the alicyclic structure-containing polymer contains a cycloalkane structure.

The number of carbon atoms included in the alicyclic structure is not particularly limited, but is normally 4 to 30, preferably 5 to 20, and more preferably 5 to 15. When the number of carbon atoms included in the alicyclic structure is within the above range, the alicyclic structure-containing polymer exhibits mechanical strength, heat resistance, and formability in a highly balanced manner, which is suitable.

The content of an alicyclic structure-containing repeating unit in the alicyclic structure-containing polymer may be appropriately selected taking account of the intended use, but is normally 30 wt% or more, preferably 50 wt% or more, and still more preferably 70 wt% or more. If the content of the alicyclic structure-containing repeating unit in the alicyclic structure-containing polymer is too low, the alicyclic structure-containing polymer may exhibit poor heat resistance, which is not preferable. A remaining repeating unit included in the alicyclic structure-containing polymer other than the alicyclic structure-containing repeating unit is not particularly limited, and is appropriately selected taking account of the intended use.

Specific examples of the alicyclic structure-containing polymer include (1) a norbornene-based polymer, (2) a monocyclic cycloolefin-based polymer, (3) a cyclic conjugated diene-based polymer, and (4) a vinyl alicyclic hydrocarbon-based polymer, and the like. Among these, a norbornene-based polymer is preferable from the viewpoint of heat resistance, mechanical strength, and the like.

### (1) Norbornene-based polymer

The term "norbornene-based polymer" used herein refers to a polymer that is obtained by polymerizing a norbornene-based monomer (i.e., a monomer that includes a norbornene skeleton). Norbornene-based polymers are roughly classified into a norbornene-based polymer obtained by ring-opening polymerization, and a norbornene-based polymer obtained by addition polymerization.

Examples of the norbornene-based polymer obtained by ring-opening polymerization include a ring-opening polymer of a norbornene-based monomer, a ring-opening polymer of a norbornene-based monomer and a monomer that can undergo ring-opening copolymerization with the norbornene-based monomer, hydrogenated products thereof, and the like.

Examples of the norbornene-based polymer obtained by addition polymerization include an addition polymer of a norbornene-based monomer, an addition polymer of a norbornene-based monomer and a monomer that can undergo addition copolymerization with the norbornene-based monomer, and the like.

Among these, a hydrogenated ring-opening polymer of a norbornene-based monomer, an addition polymer of a norbornene-based monomer and a monomer that can undergo copolymerization with the norbornene-based monomer, and a saturated norbornene-based polymer such as the hydrogenated addition polymer are preferable from the viewpoint of heat resistance, mechanical strength, and the like, and above all, a polymer having no polar group is preferable from the view point of ease of cell release. Here, the term "polar group" refers to a polar atomic group. Examples of the polar group include an amino group, a carboxyl group, a hydroxyl group, an acid anhydride group, and the like.

Examples of the norbornene-based monomer include a bicyclic norbornene-based monomer such as bicyclo[2.2.1]hept-2-ene (trivial name: norbornene), 5-methylbicyclo[2.2.1]hept-2-ene, 5,5-dimethylbicyclo[2.2.1]hept-2-ene, 5-ethylbicyclo[2.2.1]hept-2-ene, 5-ethylidenebicyclo[2.2.1]hept-2-ene, 5-vinylbicyclo[2.2.1]hept-2-ene, 5-propenylbicyclo[2.2.1]hept-2-ene, 5-methoxycarbonylbicyclo[2.2.1]hept-2-ene, 5-cyanobicyclo[2.2.1]hept-2-ene, and 5-methyl-5-methoxycarbonylbicyclo[2.2.1]hept-2-ene; a tricyclic norbornene-based monomer such as tricyclo[ 4.3.0^{1,6}.1^{2,5}]deca-3,7-diene (trivial name: dicyclopentadiene), 2-methyldicyclopentadiene, 2,3-dimethyldicyclopentadiene, and 2,3-dihydroxydicyclopentadiene; a tetracyclic norbornene-based monomer such as tetracyclo[4.4.0.1^{2,5}.1^{7,10}]-3-dodecene (tetracyclododecene), tetracyclo[4.4.0.1^{2,5}.1^{7,10}]-3-dodecene, 8-methyltetracyclo[4.4.0.1^{2,5}.1^{7,10}]-3-dodecene, 8-ethyltetracyclo[4.4.0.1^{2,5}.1^{7,10}]-3-dodecene, 8-ethylidenetetracyclo[4.4.0.1^{2,5}.1^{7,10}]-3-dodecene, 8,9-dimethyltetracyclo[4.4.0.1^{2,5}.1^{7,10}]-3-dodecene, 8-ethyl-9-methyltetracyclo[4.4.0.1^{2,5}.1^{7,10}]-3-dodecene, 8-ethylidene-9-methyltetracyclo[4.4.0.1^{2,5}.1^{7,10}]-3-dodecene, 8-methyl-8-carboxymethyltetracyclo[4.4.0.1^{2,5}.1^{7,10}]-3-dodecene,7,8-benzotricyclo[4.3.0.1^{2,5}]dec-3-ene (trivial name: methanotetrahydrofluorene (also referred to as 1,4-methano-1,4,4a,9a-tetrahydrofluorene)), 1,4-methano-8-methyl-1,4,4a,9a-tetrahydrofluorene, 1,4-methano-8-chloro-1,4,4a,9a-tetrahydrofluorene, and 1,4-methano-8-bromo-1,4,4a,9a-tetrahydrofluorene; and the like.

Examples of the additional monomer that can undergo ring-opening copolymerization with the norbornene-based monomer include a monocyclic cycloolefin-based monomer such as cyclohexene, cycloheptene, cyclooctene, 1,4-cyclohexadiene, 1,5-cyclooctadiene, 1,5-cyclodecadiene, 1,5,9-cyclododecatriene, and 1,5,9,13-cyclohexadecatetraene.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

Examples of the additional monomer that can undergo addition copolymerization with the norbornene-based monomer include an α-olefin-based monomer having 2 to 20 carbon atoms, such as ethylene, propylene, 1-butene, 1-pentene, and 1-hexene; a cycloolefin-based monomer such as cyclobutene, cyclopentene, cyclohexene, cyclooctene, and tetracyclo[9.2.1.0^{2,10}.0^{3,8}]tetradeca-3,5,7,12-tetraene (also referred to as "3a,5,6,7a-tetrahydro-4,7-methano-1H-indene); a non-conjugated diene-based monomer such as 1,4-hexadiene, 4-methyl-1,4-hexadiene, 5-methyl-1,4-hexadiene, and 1,7-octadiene; and the like.

Among these, an α-olefin-based monomer is preferable, and ethylene is more preferable as the monomer that can undergo addition copolymerization with the norbornene-based monomer.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

A ring-opening polymer of a norbornene-based monomer, or a ring-opening polymer of a norbornene-based monomer and a monomer that can undergo ring-opening copolymerization with the norbornene-based monomer, may be obtained by polymerizing the monomer component in the presence of a known ring-opening polymerization catalyst. Examples of the usable ring-opening polymerization catalyst include a catalyst that includes a halide of a metal (e.g., ruthenium or osmium), a nitrate or an acetylacetone compound, and a reducing agent, or alternatively a catalyst that includes a halide or an acetylacetone compound of a metal (e.g., titanium, zirconium, tungsten, or molybdenum), and an organoaluminum compound.

A hydrogenated ring-opening polymer of a norbornene-based monomer may normally be obtained by adding a known hydrogenation catalyst that includes a transition metal (e.g., nickel or palladium) to a solution including the ring-opening polymer, and hydrogenating the carbon-carbon unsaturated bonds of the ring-opening polymer.

An addition polymer of a norbornene-based monomer, or an addition polymer of a norbornene-based monomer and a monomer that can undergo addition copolymerization with the norbornene-based monomer, may be obtained by polymerizing the monomer component in the presence of a known addition polymerization catalyst. Examples of the usable addition polymerization catalyst include a catalyst that includes a titanium, zirconium, or vanadium compound and an organoaluminum compound.

### (2) Monocyclic cycloolefin-based polymer

Examples of the monocyclic cycloolefin-based polymer include an addition polymer of a monocyclic cycloolefin-based monomer (e.g., cyclohexene, cycloheptene, or cyclooctene), and the like.

### (3) Cyclic conjugated diene-based polymer

Examples of the cyclic conjugated diene-based polymer include a 1,2-addition polymer or a 1,4-addition polymer of a cyclic conjugated diene-based monomer (e.g., cyclopentadiene or cyclohexadiene), a hydrogenated product thereof, and the like.

### (4) Vinyl alicyclic hydrocarbon polymer

Examples of the vinyl alicyclic hydrocarbon polymer include a polymer of a vinyl alicyclic hydrocarbon-based monomer (e.g., vinylcyclohexene or vinylcyclohexane), and a hydrogenated product thereof; a hydrogenated product obtained by hydrogenating the aromatic ring of a polymer of a vinyl aromatic-based monomer (e.g., styrene or α-methylstyrene); and the like.

The vinyl alicyclic hydrocarbon polymer may be a copolymer of the above monomer and an additional monomer that can undergo copolymerization with the above monomer.

These alicyclic structure-containing polymers may be used either alone or in combination.

The molecular weight of the alicyclic structure-containing polymer is not particularly limited. The polystyrene-equivalent weight average molecular weight of the alicyclic structure-containing polymer determined by gel permeation chromatography using a cyclohexane solution (or a toluene solution when the polymer does not dissolve in cyclohexane) is normally 5,000 or more, preferably 5,000 to 500,000, more preferably 8,000 to 200,000, and particularly preferably 10,000 to 100,000. When the weight average molecular weight of the alicyclic structure-containing polymer is within the above range, the alicyclic structure-containing polymer exhibits mechanical strength and formability in a highly balanced manner, which is suitable.

The glass transition temperature of the alicyclic structure-containing polymer may be appropriately selected taking account of the intended use. The glass transition temperature of the alicyclic structure-containing polymer is normally 50 to 300°C, preferably 100 to 280°C, more preferably 115 to 250°C, and still more preferably 130 to 200°C. When the glass transition temperature of the alicyclic structure-containing polymer is within the above range, the alicyclic structure-containing polymer exhibits heat resistance and formability in a highly balanced manner, which is suitable.

Note that the glass transition temperature of the alicyclic structure-containing polymer refers to a value measured in accordance with JIS K 7121.

An additive that is normally used for a thermoplastic resin material, such as a soft polymer, an antioxidant, a UV absorber, a light stabilizer, a near-infrared absorber, a release agent, a coloring agent such as dye and pigment, a plasticizer, an antistatic agent, and a fluorescent whitening agent, may be added to the alicyclic structure-containing polymer in an amount that is normally employed.

In addition, an additional polymer other than the soft polymer (hereinafter referred to as "additional polymer") may be mixed with the alicyclic structure-containing polymer. The additional polymer is normally mixed with the alicyclic structure-containing polymer in a ratio of 200 parts by weight or less, preferably 150 parts by weight or less, and more preferably 100 parts by weight or less, based on 100 parts by weight of the alicyclic structure-containing polymer.

If the ratio of the additive or the additional polymer is too large based on the alicyclic structure-containing polymer, the cells may be suspended to only a small extent. Therefore, it is preferable to add (mix) the additive and the additional polymer within such a range that the properties of the alicyclic structure-containing polymer are not impaired.

The additive and the additional polymer may be mixed with the alicyclic structure-containing polymer using an arbitrary method as long as the additive is sufficiently dispersed in the polymer. The additive and the additional polymer may be added in an arbitrary order. Examples of the mixing method include a method that mixes (kneads) the resin in a molten state using a mixer, a single-screw kneader, a twin-screw kneader, a roll, a Brabender, an extruder, or the like, a method that dissolves the resin in an appropriate solvent to effect dispersion, and removes the solvent using a coagulation method, a casting method, or a direct drying method, and the like. When the resin is mixed (kneaded) using a twin-screw kneader, the resulting mixture (kneaded product) is normally extruded in the shape of a rod in a molten state, cut to have an appropriate length using a strand cutter, and pelletized for use.

A forming method that is used when forming the alicyclic structure-containing polymer culture vessel may be arbitrarily selected taking account of the shape of the culture vessel. Specific examples of the forming method include an injection forming method, an extrusion method, a cast forming method, an inflation forming method, a blow forming method, a vacuum forming method, a press forming method, a compression forming method, a rotational forming method, a calendering method, a roll forming method, a cutting method, a spinning method, and the like. Note that these methods may be used in combination, and a post-treatment such as stretching may optionally be performed after forming.

For the alicyclic structure-containing polymer culture vessel used in connection with the invention, a vessel is enough in which at least a surface in contact with cells is made of an alicyclic structure-containing polymer, and the whole vessel does not have to be made of the alicyclic structure-containing polymer. Furthermore, it may be a vessel containing the alicyclic structure-containing polymer as a part of component members, a vessel composed entirely of the alicyclic structure-containing polymer, or a vessel composed of a laminate of the alicyclic structure-containing polymer formed article and an additional polymer formed article.

The shape of the alicyclic structure-containing polymer culture vessel in contact with cells is not particularly limited, it may have a plate-like shape, a sheet-like shape and the like, and its surface may be either flat or irregular. Specific examples of the shape of the vessel include dish, plate, bag, tube, scaffold, cup, jar fermenter and the like.

In the present invention, the alicyclic structure-containing polymer culture vessel is preferably sterilized.

The sterilization method is not particularly limited. The sterilization method may be selected from sterilization methods that are normally employed in the medical field, including a heating method such as a high-pressure steam method and a dry heat method, a radiation method that applies radiation such as γ-rays or an electron beam, and an irradiation method that applies high-frequency waves, a gas method that brings a gas such as ethylene oxide gas (EOG) into contact with the sterilization target, and a filtration method that utilizes a sterilization filter, taking account of the shape of the formed article and the cells to be cultured. It is preferable to use a gas method since a change in surface state occurs to only a small extent.

The surface of the formed article may also be subjected to a treatment (e.g., plasma treatment, corona discharge treatment, ozone treatment, and UV irradiation treatment) other than the sterilization treatment that is normally used for a culture vessel. Note that, to suppress an increase in cost due to the surface treatment operation, prevent a situation in which cleanliness is impaired due to partial decomposition of the surface of the formed article during the surface treatment, and prevent a decrease in the releasability of the cells, the water contact angle of the bottom surface in contact with the cells in the alicyclic structure-containing polymer culture vessel is preferably 85° to 110°, more preferably 85° to 105°, and particularly preferably 85° to 100°. Note that the term "water contact angle" used herein refers to a value obtained by cutting the bottom surface of the dish using a circle cutter having a diameter of 30 mm to prepare a specimen, measuring the radius r and the height h of a liquid droplet at the center of the specimen and the four vertices of a 20×20 mm square formed around the center of the specimen (i.e. at five measurement points) using a known automatic contact angle meter (e.g. "LCD-400S" manufactured by Kyowa Interface Science Co., Ltd.), and calculating the value θ using the expressions "tanθ1=h/r" and "0=2arctan(h/r)" (θ/2 method).

For the cultured cells to suspend in the liquid medium, the cells adhering to the culture vessel should be released. Since adherent cells cultured in an alicyclic structure-containing polymer culture vessel are not adhered in an extended state like adherent cells cultured in a polystyrene vessel but arc adhered in a state of maintaining a relatively spherical shape, the cells can be easily released by applying a small force. Hence, in the present invention, the cells are released using only liquid flow. Upon releasing the cells, neither a proteinase nor an instrument such as a scraper that directly provides any force to the cells are required.

The liquid flow can be generated by an action applied to cells cultured by moving a liquid medium or a liquid constituting a liquid medium. Specific examples thereof include methods for intentionally applying force to a liquid in a cell vessel, such as inhalation and discharge by pipetting operation or the like, vibration and agitation by vortexing operation, ultrasonication or the like, and circulation using a small pump or the like.

The liquid for causing liquid flow may be not only the liquid medium in the culture vessel used for culture but also a liquid component constituting a medium, such as saline, another medium, a medium other than that used for culture, a buffer, and a solution in which some components of the medium is dissolved in a buffer. When using a medium other than the liquid medium in the culture vessel used for the culture, a part or whole of the original medium present in the culture vessel may be removed, or naturally the medium may be added in the presence of the original medium.

The method according to the present invention may be applied to liquid operation by pipetting through a plurality of channels using a multichannel head of an automatic dispenser or through a single channel, and also may be applied to liquid operation by pipetting through an arm-type robot or a humanoid robot. In addition, it may be applied to a cell culture operation in an isolator for cell culture.

### EXAMPLES

The invention is further described below by way of examples. Note that the invention is not limited to the following examples.

### [Example 1]

A plate-shaped culture dish having a diameter of 35 mm was obtained (hereinafter referred to as "1060R dish") by an injection forming method using a hydrogenated norbornene-based ring-opening polymer [ZEONOR (registered trademark) 1060R (manufactured by Zeon Corporation)] as an alicyclic structure-containing polymer.

Then, the 1060R dish was subjected to ethylene oxide sterilization. The water contact angle of the bottom surface (side in contact with the cells) of the 1060R dish was 90°.

CHO cells were seeded onto a Ham's medium containing 10% fetal bovine serum as a liquid medium at a cell density of 2.878×10⁴ cells/mL using the 1060R dish as a culture vessel, and cultured in a 5% CO₂ atmosphere at 37°C until they reached a confluent state. The cells were collected and subcultured twice every 3 days under the same conditions. For the subculture, the culture solution in the culture vessel was pipetted 30 times (0.5 mL), and force of the liquid flow was applied to the whole bottom surface of the culture vessel to release the cells. The culture solution having the suspended cells was diluted by 10 times and subcultured. Ham's medium was used for dilution.

At a time when two subcultures and three-day culture had been finished, the culture solution was pipetted 30 times (0.5 mL) to release the cells and the cell count was measured in accordance with the following method.

### (Measurement of cell count)

With regard to the cells in a suspended state, the culture supernatant was used as a sample, the sample was stained with trypan blue to distinguish the viable cells and the dead cells, and the cell count was measured.

On the other hand, the cells adhering to the bottom surface of the 1060R dish were washed with physiological saline, then released from the 1060R dish by trypsinization, then stained with trypan blue to distinguish the viable cells and the dead cells, and the cell count was measured.

### [Reference Example 1]

Cells were cultured and the cell count was measured in the same manner as in Example 1, except that a polystyrene dish [Falcon (registered trademark) dish (manufactured by Becton, Dickinson and Company, model number: 353001)] was used instead of the 790R dish and trypsinization was carried out before pipetting.

Figure 1 shows the total number of viable cells in the 1060R dish in Example 1 when the total number of the viable cells in the polystyrene dish in Reference Example 1 is taken as 1.

The results indicate that the CHO cells cultured in the 1060R dish are easily released from the culture vessel by pipetting, and thus the cells are steadily increased by subculture, and can be subcultured to the same degree as in a conventional manner i.e. a method in which the cells are cultured in a polystyrene dish, released by trypsinization, and subcultured.

### [Comparative Example 1]

Cells were subcultured in the same manner as Example 1 except that a polystyrene dish was used instead of the 1060R dish, but most of the cells were not released by pipetting, most of the cells could not be secured in the first subculture, and no viable cell could be confirmed in the second subculture.

## Claims

1. A method for culturing adherent cells, culturing the adherent cells in a liquid medium in an alicyclic structure-containing polymer culture vessel, releasing the cells from the culture vessel by liquid flow without adding a protease, and suspending the cultured cells in a liquid medium.

2. The method for culturing adherent cells according to claim 1, wherein the adherent cells are CHO cells.

3. The method for culturing adherent cells according to claim 1 or 2, wherein a water contact angle of the bottom surface in contact with the cells in the alicyclic structure-containing polymer culture vessel is 85° to 110°.
